Europäisches Patentamt

European Patent Office

Office européen des brevets

(19)

(11) Veröffentlichungsnummer: **0 130 356**
**A1**

(12) **EUROPÄISCHE PATENTANMELDUNG**

(21) Anmeldenummer: **84105999.1**

(22) Anmeldetag: **25.05.84**

(51) Int. Cl.⁴: **A 61 F 13/20,** A 61 L 15/00

(30) Priorität: **03.06.83 DE 3320218**

(43) Veröffentlichungstag der Anmeldung: **09.01.85**
**Patentblatt 85/2**

(84) Benannte Vertragsstaaten: **AT BE CH DE FR GB IT LI NL SE**

(71) Anmelder: **Henkel Kommanditgesellschaft auf Aktien, Postfach 1100 Henkelstrasse 67, D-4000 Düsseldorf-Holthausen (DE)**

(72) Erfinder: **Sustmann, Scarlet, Dr., Am Nachtigallenwäldchen 34, D-4060 Viersen 12 (DE)**

(54) Tampons mit Schutzwirkung vor vaginalen Infekten und Verfahren zu deren Herstellung.

(57) Die Erfindung betrifft Tampons mit Schutzwirkung vor vaginalen Infekten, die dadurch gekennzeichnet sind, daß der Tamponkörper mit einem Puffer präpariert ist.

Die Erfindung betrifft weiterhin ein Verfahren zur Herstellung der Tampons.

EP 0 130 356 A1

4ooo Düsseldorf, den 30.5.1983
Henkelstr. 67

D 6625 EP

0130356

HENKEL KGaA
ZR-FE/Patente
v.Kr

- Tampons mit Schutzwirkung vor vaginalen Infekten
und Verfahren zu deren Herstellung

---

Der pH-Wert der Vagina liegt im Falle einer ungestörten Vaginalflora im sauren Bereich (pH 3,5 - 5). Bei Störungen der natürlichen Flora, z.B. infolge Pilzbefall, Trichomenaden etc. oder zum Teil auch bei Einnahme von Kontrazeptiva, verschiebt er sich zu neutralen bis alkalischen Werten. Damit wird aber auch einer unnatürlichen Bakterienbesiedlung und damit einer Anfälligkeit gegenüber Erkrankungen Vorschub geleistet.

Der pH-Wert der Menstruationsflüssigkeit liegt im allgemeinen im alkalischen Bereich (pH größer 7.).

Normalerweise fließt die Menstrualflüssigkeit nach außen ab. Bei Verwendung üblicher Tampons wird aber die Menstrualflüssigkeit mit ihrer relativ hohen Pufferkapazität im Vaginalbereich festgehalten. Dies führt, was den pH-Wert betrifft, zu unphysiologischen Bedingungen und kann das Wachstum von Bakterien aufgrund der pH-Verschiebung ungünstig beeinflussen.

In Kenntnis dieser Tatsache wurden bereits Tampons vorgeschlagen, die durch eine saure Präparationsauflage oder sonstige Zusätze den pH-Wert positiv beeinflussen sollen.

So z.B. in der DT 309 575, die die Aufrechterhaltung eines pH-Wertes von 4.0 durch eine Imprägnierung der Tampons mit Glycogen, Zucker, Döderlein-Bakterien, Östrogenen usw. anstrebt, also solchen Substanzen, aus denen letztlich durch Lactobac.acidoph. Milchsäure gebildet wird, bzw. die Imprägnierung mit Milchsäure direkt.

Patentanmeldung  D 6625 EP

0130356

HENKEL KGaA
ZR-FE/Patente

Zur Regenerierung des physiologischen pH-Wertes sind auch Zäpfchen mit tiefgefriergetrockneten Kulturen des Lactobac.acidoph. im Handel (WFA Westfalia Arzneimittel GmbH in Med.Klin. 76, 28 (1981)). Die Zusätze, die durch Lactobac.acid. eine Säuerung erzeugen sollen, haben jedoch den Nachteil, daß gerade während der Menstruation, wenn der pH-Wert der Vagina im alkalischen Bereich liegt, diese Bakterien in ihrem Wachstum stark gehemmt sind, so daß die Erzeugung von Milchsäure unter diesen Bedingungen nicht im wesentlichen Ausmaß stattfinden kann.

Eine andere Veröffentlichung (US-PS 3 995 636) beschreibt einen Tampon aus polymerem Schaum, der mit Citronensäure und $NaHCO_3$ imprägniert wurde.

Die oben genannten Imprägnierungsverfahren haben noch einen anderen wesentlichen Nachteil. Da die Menstruationsflüssigkeit eine hohe Pufferwirkung besitzt, ist bei den mit einem Tampon anzubietenden Säuremengen die Wirkung bereits bei geringem Blutanfall erschöpft.

Zudem ist der Fremdzusatz von Säure sicherlich problematisch, da es bei Überdosierung der Säure einerseits oder hoher Empfindlichkeit der Haut gegen Säure andererseits zu Reizungen kommen kann.

Den genannten Nachteilen wird dadurch begegnet, daß man die Tampons nicht mit Säure imprägniert, sondern ein Puffersystem verwendet.

Demgemäß betrifft die vorliegende Erfindung Tampons mit Schutzwirkung vor vaginalen Infekten, die dadurch gekennzeichnet sind, daß der Tamponkörper mit einem Puffer präpariert ist, sowie Verfahren zu deren Herstellung.

Patentanmeldung    D 6625 EP

Grundsätzlich kann für die erfindungsgemäßen Tampons jedes physiologisch arbeitende Puffersystem eingesetzt werden. Im vorliegenden Verfahren werden Viskosefasern mit einer Citratpufferauflage versehen. Dies geschieht dadurch, daß man dem wässrigen Medium bei der Herstellung der Viskosefaser Citratpuffer in einer Menge zusetzt, die die Einstellung des gewünschten Faser-pH-Wertes bewirkt. So wurden beispielsweise Fasern mit einem Faser-pH-Wert von 4,65 bei einem Flotteverhältnis 1 : 50 hergestellt.

Die erfindungsgemäßen Tampons können auch dadurch hergestellt werden, daß der Citratpuffer auf einen fertigen Tamponkörper aufgebracht wird. Dies geschieht beispielsweise dadurch, daß der fertige Tamponkörper mit wässriger Citratpuffer-Lösung getränkt und anschließend gefriergetrocknet wird.

Die Auflage kann zwischen 0,05 % bis prinzipiell größer 0,5 % bezogen auf das Fasergewicht,betragen. Bevorzugte Werte liegen zwischen 0,1 und 0,3 %. Grenzen bezüglich der Auflage des Citratpuffers sind dadurch gegeben, daß die Verarbeitbarkeit des Fasermaterials mit steigendem Anteil an Pufferlösung in der Flotte schlechter wird; in gleicher Richtung nimmt auch das Saugvermögen des fertigen Tampons ab.

Klinikversuche mit Citrat-gepufferten Tampons (Faser-pH-Wert 4,65) ergaben bei einer mittleren Blutaufnahme von 6,1 ml im Vergleich zu unbehandelten Tampons eine Reduzierung des pH-Wertes um 0,25 Einheiten (unbehandelte Tampons: pH = 8,02; gepufferte Tampons: pH = 7,67).

Bei einer geringen Blutaufnahme (ca. 2,0 ml) ist der Effekt ausgeprägter. Man erhält eine Differenz von 1,6 pH-Einheiten (7,7 bzw. 6,1).

Patentanmeldung D 6625 EP

HENKEL KGaA
ZR-FE/Patente

Im Falle der Aufnahme von 8 ml und mehr Blut, ging die Wirkung bei einer Citrat-Puffer-Auflage von 0,1 % verloren. Um auch bei derart großen Blutmengen den gewünschten Effekt, nämlich die Absenkung des pH-Wertes in den sauren Bereich, zu erzielen, muß die Pufferauflage erhöht werden.

Wie in Kliniktests gezeigt werden konnte, ist durch die Präparation von Viskosefaser oder fertigen Tampons mit Citrat-Puffer ein positiver Einfluß auf den pH-Wert im Vaginalbereich zu erzielen. Besonders bemerkenswert ist, daß eine Absenkung des pH-Wertes in den sauren Bereich (pH 4 - 6) auch bei größeren Blutmengen und insbesondere nach langer Tragedauer der Tampons (8 - 10 Stunden) möglich ist.

Kolposkopische Untersuchungen der Vaginalhaut nach der Anwendung der erfindungsgemäßen Tampons zeigten keinerlei schädigende Einflüsse.

Die Ausführungsformen der erfindungsgemäßen Tampons wie die Verfahren zu ihrer Herstellung werden durch die folgenden Beispiele erläutert.

Beispiel 1
Herstellung von Tampons aus mit Citratpuffer beaufschlagter Viskosefaser

1 kg Normalviskosefasern (2,8 dtex/3o mm, matt. unaviviert) wurde mit 10 l Citratpuffer-Avivage 30 min. bei 50°C behandelt. Die Fasern wurden auf eine Feuchte von ca 200% abgepreßt und im Umlufttrockenschrank 4 Std. bei 105°C getrocknet. Die Bestimmung der Avivageauflage nach der Ethanolextraktionsmethode lieferte einen Wert von 0,17%.

0130356

HENKEL KGaA
ZR-FE/Patente

Die Citratpuffer-Avivage hatte folgende Zusammensetzung:

117,6 g Zitronensäure

44,8 g Ätznatron

43,4 g 37 %ige Salzsäure

7.0 g Stearinsäureethoxylat (4 - 6 Mol
Ethylenoxid/Mol Stearinsäure) mit destilliertem Wasser auf 10 l aufgefüllt.

Zum Vergleich:

1 kg Normalviskosefasern (2,8 dtex/30 mm, matt, unaviviert) wurde mit 10 l Avivage 30 min. lang bei 50°C behandelt. Die Fasern wurden auf ca 200 % Feuchte abgepresst und im Umlufttrockenschrank 4 Std. bei 105°C getrocknet. Die Bestimmung der Avivageauflage nach der Ethanolextraktionsmethode lieferte einen Wert von 0,09 %.

Die verwendete Avivage hatte folgende Zusammensetzung:
7,0 g Stearinsäureethoxylat
(4 - 6 Mol Ethylenoxid/Mol Stearinsäure)
mit destilliertem Wasser auf 10 l aufgefüllt.

Die Bestimmung des Faser-pH-Wertes erfolgte nach DIN 54275 bei einem Flottenverhältnis von 1 : 50.

Für die nicht mit Puffer präparierte Watte wurde nach dieser Normvorschrift ein pH-Wert von 6,45 erhalten, während die mit Citratpuffer behandelte Faser einen pH-Wert von 4,65 ergab.

Die Fasern wurden nach an sich üblichen Krempel- und Kalandrierverfahren zu Wattband mit einem Flächengewicht von 640 g/m² und einer Breite von 90 mm verarbeitet. Von diesem Watteband wurden 40 mm lange Abschnitte abgetrennt. In Längsrichtung der Abschnitte wurde ein Rückholband aufgenäht.

Die Verpressung der Wattebandabschnitte wurde in axialer und radialer Richtung vorgenommen (Tampax-Verfahren), so daß zylindrische Körper mit runder Kopfanformung und einem Gewicht von ca 2,3 g resultierten. Die Abmessungen im Vergleich zu ebenso hergestellten Tampons aus unpräparierter Watte sind Tabelle 1 (s. Beispiel 3) zu entnehmen.

Selbstverständlich können auch Tampons nach anderen Verfahren, z.B. dem Wickelverfahren, hergestellt werden.

Beispiel 2
Tamponherstellung aus unpräparierten Fasern und Aufbringung des Puffers auf den fertigen Tampon

Tampons aus normalen Viskosefasern, z.B. mit dtex 3,6/-30-40 mm, wurden nach dem in Beispiel 1 beschriebenen Verfahren hergestellt und in ein Hülsenpaar eingebracht. Die Hülsen bestanden entweder aus Plastik (z.B. Polypropylen) oder wurden aus Papierbahnen in der Weise gewickelt, daß die Innenseite hydrophob war, um eine Aufnahme von Flüssigkeit bei der Präparation zu vermeiden.
Als geeignet erwies sich z.B. Polyvinylidenchlorid- oder Polyethylen-beschichtetes Papier.

Der Tampon wurde in die Außenhülse so eingeführt, daß am Kopfende ein Raum für ca 0,25 bis 3 ml Flüssigkeit gebildet wurde.

Die Pufferlösung wurde beispielsweise aus 2 M Natronlauge und 2 M Zitronensäure so hergestellt, daß ein pH von ca 3,0 bis 4,0 resultierte. Zur Erreichung eines pH-Wertes von ca 3,1 mischte man z.B. 7 ml 2 M Natronlauge mit 10 ml 2 M Zitronensäure und füllte mit Wasser auf 100 ml auf.

1 - 2 ml der Pufferlösung wurden auf den Tamponkopf aufgegeben, dabei verteilte sich die Flüssigkeit über den gesamten Tampon.

Anschließend wurde mit Heißluft oder nach üblichen Gefriertrocknungsverfahren der imprägnierte Tampon getrocknet. Dabei zog er sich so weit zusammen, daß er bei Anwendung mit der Innenhülse leicht aus der Außenhülse herausgeschoben werden konnte.

Selbstverständlich können nach diesen Verfahren beliebige Pufferlösungen oder aber auch andere Wirkstoffe allein oder in Kombination mit Puffersubstanzen aufgebracht werden.

Beispiel 3
Bestimmung der Saugkapazität

Die Saugfähigkeit der nach Beispiel 1 bzw. Beispiel 2 hergestellten Tampons wurde in einer künstlichen Vagina (Syngina) mit Wasser als Testflüssigkeit bestimmt. Dieses Gerät bestand aus einem Plastikrohr (Innendurchmesser 35 mm), in dem eine elastische Folie gespannt war. Nach Einschieben des Tampons wurde zwischen Rohrwand und Folie ein definierter Druck (30 mbar) mit Preßluft aufgebaut. Mit einer Tropfgeschwindigkeit von 3,5 bis 4,5 ml/min. wurde das gefärbte Wasser aus einer Meßbürette bis zum Durchdringen des ersten Tropfens am unteren Ende des Tampons auf den Tamponkopf aufgegeben. Die durch den Tampon aufgenommene Flüssigkeitsmenge wurde an der Bürette abgelesen. Die Messung erfolgte nicht isobar, d.h. durch die Ausdehnung des
Tampons nahm der Druck kontinuierlich um ca 7 mbar zu.

Die Abmessungen der getesteten Tampons sowie die Ergebnisse der Saugkapazitäts-Messung sind der nachfolgenden Tabelle 1 zu entnehmen.

Tabelle 1

Abmessungen der Tampons und ihre Saugkapazität

| Tampon | Gewicht (g) | Länge (mm) | Durchmesser (mm) | Saugkapazität | |
|---|---|---|---|---|---|
| | | | | ml $H_2O$/ Tampon | ml/ g Watte |
| mit Puffer | 2,3 | 39,7 | 12,4 | 7,7 | 3,35 |
| ohne Puffer | 2,3 | 39,5 | 12,3 | 7,8 | 3,39 |

Ergebnis:
Die Saugfähigkeit der Tampons wird durch die Pufferauflage praktisch nicht verändert.

Beispiel 4
Bestimmung des pH-Wertes getragener, mit Menstrualblut
getränkter Tampons im in-vivo-Test

Je 5 Tampons, einzeln gewogen und verpackt, wurden an
13 Probandinnen ausgegeben (65 Tampons).
Nach Gebrauch wurden die Tampons an das klinische Labor
zurückgegeben, zur Bestimmung der aufgenommenen Menstrualblut-Menge gewogen und der Faser-pH-Wert der
Tampons an den mit Blut durchsetzten Stellen mit einer
Vaginalelektrode der Firma Dr.W, Ingold KG gemessen.

0130356
Patentanmeldung D 6625 EP

HENKEL KGaA
ZR-FE/Patente

Die mittleren pH-Werte der Tampons sind nachstehender Tabelle 2 zu entnehmen.

Tabelle 2

Tampon-pH-Wert Citratpuffer-beaufschlagter Tampons nach der Benutzung in Abhängigkeit von der Menstrualblutmenge

| Tampon | aufgenommene Menstrualblutmenge (ml) | | |
|---|---|---|---|
| | $< 4,1$ | $4,1 - 7,5$ | $7,5$ |
| unbehandelt | 7,71 | 8,7 | - |
| mit Citratpuffer beaufschlagt | 5.95 | 7,8 | 8,9 |

Ergebnis:

Die Tragezeiten betrugen bis zu 10 Stunden. Eine Abhängigkeit des pH-Wertes von der Tragezeit wurde in den einzelnen Gruppen nicht beobachtet. Der pH-Wert der mit Citratpuffer beaufschlagten Tampons liegt deutlich niedriger als der der unbehandelten Tampons bei gleicher Menge aufgenommenen Blutes.

Vor und nach der Benutzung von 5 Tampons mit Pufferauflage erfolgte eine kolposkopische Untersuchung der Vaginalhaut der Probandinnen. Es wurden keinerlei Reizungen oder ähnliches festgetellt.

Patentanmeldung   D 6625 EP

0130356
HENKEL KGaA
ZR-FE/Patente

Patentansprüche

1. Tampons mit Schutzwirkung vor vaginalen Infekten, dadurch gekennzeichnet, daß der Tamponkörper mit einem Puffer präpariert ist.

2. Tampons nach Anspruch 1, dadurch gekennzeichnet, daß das Kapazitätsoptimum des Puffers vorzugsweise bei pH 4,0 bis pH 6,0 liegt.

3. Tampons nach Ansprüchen 1 und 2, dadurch gekennzeichnet, daß der Puffer ein Citratpuffer ist.

4. Verfahren zur Herstellung von Tampons nach Ansprüchen 1 - 3, dadurch gekennzeichnet, daß Viskosefasern zur Herstellung von Tampons mit einer Pufferauflage nach Ansprüchen 2 und 3 versehen und danach zu Tampons verarbeitet werden.

5. Verfahren zur Herstellung von Tampons nach Ansprüchen 1 - 3, dadurch gekennzeichnet, daß der fertige Tamponkörper mit Puffer nach Ansprüchen 2 und 3 beaufschlagt wird.

6. Verfahren nach Anspruch 5, dadurch gekennzeichnet, daß dies durch Aufbringen einer wässrigen Lösung des Citratpuffers und anschließende Gefriertrocknung geschieht.

7. Verfahren nach Ansprüchen 4 - 6, dadurch gekennzeichnet, daß die Citratpuffer-Auflage zwischen 0,05 und größer 0,5 % beträgt.

8. Verfahren nach Ansprüchen 4 - 7, dadurch gekennzeichnet, daß die Auflage 0,1 - 0,3 % an Citratpuffer beträgt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X,A | US-A-3 794 034  (JONES) <br> * Anspruch 1 * <br><br> --- | 1-3 | A 61 F  13/20 <br> A 61 L  15/00 |
| X | CH-A- 335 382  (AB WALLCO) <br> * Ganzes Dokument * <br><br> --- | 1 | |
| A | DE-C- 916 572  (FRIEDRICH) <br><br> --- | | |
| A | DE-B-1 166 412  (WISCONSIN ALUMNI RESEARCH FOUNDATION) <br><br> --- | | |
| A | DE-A-2 309 575  (WENDERLEIN) <br><br> --- | | |
| A | FR-A-2 513 875  (UNIVERSITY OF DELAWARE) <br><br> --- | | **RECHERCHIERTE SACHGEBIETE (Int. Cl. ³)** |
| A | US-A-3 067 745  (BURGENI et al.) <br><br> --- | | A 61 F  13/00 <br> A 61 L  15/00 |
| D,A | US-A-3 995 636  (MURRAY et al.) <br><br> ----- | | |

Der vorliegende Recherchenbericht wurde für alle Patentansprüche erstellt.

| Recherchenort | Abschlußdatum der Recherche | Prüfer |
|---|---|---|
| BERLIN | 05-09-1984 | KANAL P K |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82